# EUROPÄISCHE PATENTSCHRIFT

(11) **EP 3 100 749 B1**
(45) Veröffentlichungstag und Bekanntmachung des Hinweises auf die Patenterteilung: **07.11.2018**
(21) Anmeldenummer: 15170877.3
(22) Anmeldetag: 05.06.2015
(51) Int. Cl.: A61M 1/16

(54) **HÄMODIALYSEGERÄT**
HAEMODIALYSIS DEVICE
APPAREIL D'HÉMODIALYSE

(43) Veröffentlichungstag der Anmeldung: 07.12.2016
(73) Patentinhaber: D_MED Consulting AG, 47269 Duisburg (DE)
(72) Erfinder: Biermann, Frank, 22455 Hamburg (DE); Breuch, Gerd, 53844 Troisdorf (DE); Yanagimoto, Yoji, 3080 Tervuren (Moorsel) (BE); Breuch, Julius, 22761 Hamburg (DE); Holzschuh, Robert, 22301 Hamburg (DE)
(74) Vertreter: Patentanwälte ter Smitten Eberlein-Van Hoof Rütten Partnerschaftsgesellschaft mbB

(56) Entgegenhaltungen:
- EP-A1- 2 623 188
- WO-A1-2014/090746
- US-A- 3 528 550
- US-A- 3 598 727
- US-A- 4 202 760
- US-A- 4 293 409
- US-A1- 2003 212 314

## Beschreibung

Die Erfindung bezieht sich auf ein Hämodialysegerät für die Durchführung einer Blutreinigung an einem Patienten. In einem Hämodialysegerät wird zur Durchführung einer Hämodialyse in einem Dialyseflüssigkeits-Strang eine Dialyseflüssigkeit generiert, die einem Dialysator durch eine Dialyseflüssigkeits-Pumpe zugeführt wird, in dem die Dialyseflüssigkeit durch eine Dialysemembran getrennt an Patientenblut vorbeifließt.

Die Dialyseflüssigkeit wird in dem Dialyseflüssigkeits-Strang in der Regel durch Mischen von Dialysewasser, das von einer Dialysewasser-Quelle kommt, und einer oder mehrerer Konzentratflüssigkeiten generiert. Die Konzentratflüssigkeiten sind beispielsweise eine Bikarbonat-Lösung einerseits und ein Säurekonzentrat andererseits.

Aus EP 2 666 491 A1 ist ein Hämodialysegerät mit einem Dialyseflüssigkeits-Strang bekannt, der eine Dialyseflüssigkeits-Pumpe aufweist, die die Dialyseflüssigkeit aus einer Hauptleitung zu dem Dialysator pumpt. Für eine exakte Zudosierung der Konzentratflüssigkeit in das Dialysewasser ist stromabwärts des betreffenden Konzentratflüssigkeits- Behälters und vor einer Konzentratflüssigkeits-Einmündung eine weitere Pumpe vorgesehen, die von einer Gerätesteuerung entsprechend gesteuert wird. Wenn mehrere Konzentratflüssigkeiten zur Einleitung in das Dialysewasser vorgesehen sind, was in der Praxis die Regel ist, ist in jeder Konzentrateinleitungs-Anordnung bzw. jedem Konzentratflüssigkeits-Behälter jeweils eine eigene Pumpe zugeordnet. Die Konzentratflüssigkeits-Pumpen müssen sehr zuverlässig und sehr genau arbeiten, so dass die Konzentrat-Flüssigkeitspumpen relativ teure Komponenten sind.

Aus US 4293409 A ist ein Hämodialysegerät bekannt, das im Dialyseflüssigkeits-Strang vor dem Dialysator eine einzige Dialyseflüssigkeitspumpe aufweist. Das Mischverhältnis zwischen dem Dialysewasser und der Konzentratflüssigkeit wird über ein steuerbares Ventil im Einleitungsstrang der Konzentratflüssigkeit geregelt.

Aus US 3528550 A ist ein ähnliches Hämodialysegerät bekannt, bei dem der Zustrom der Konzentratflüssigkeit jedoch nicht geregelt wird.

Aufgabe der Erfindung ist es demgegenüber, ein Hämodialysegerät mit einem einfach und preiswert aufgebauten Dialyseflüssigkeits-Strang und ein Verfahren zur Generierung von Dialysewasser mit einem derartigen Hämodialysegerät zu schaffen, bei dem die generierte Dialyseflüssigkeit von konstanter Qualität ist.

Diese Aufgabe wird erfindungsgemäß gelöst mit einem Hämodialysegerät mit den Merkmalen des Anspruchs 1 beziehungsweise mit einem Verfahren mit den Verfahrensschritten des Anspruchs 11 zur Generierung einer Dialyseflüssigkeit in dem Hämodialysegerät, das die Vorrichtungsmerkmale des Anspruchs 1 aufweist.

Bei dem erfindungsgemäßen Hämodialysegerät weist die Konzentrateinleitungs-Anordnung einen Konzentratflüssigkeits-Behälter mit der Konzentratflüssigkeit, eine Einmündung, an der eine von dem Konzentratflüssigkeits-Behälter kommende Einleitungsleitung in die Hauptleitung mündet, und ein schaltbares Konzentrat-Ventil auf. Die Konzentrateinleitungs-Anordnung weist aber in dem Pfad zwischen dem Konzentratflüssigkeits-Behälter und der Einmündung keine Pumpe auf. Die Konzentrateinleitungs-Anordnung ist also passiv ausgebildet.

In der Hauptleitung des Dialyseflüssigkeits-Strangs ist fluidisch zwischen der Konzentratflüssigkeits-Einmündung und der Dialyseflüssigkeitspumpe eine separate Dosierpumpe vorgesehen, die fluidisch in Reihe mit der Dialyseflüssigkeitspumpe angeordnet ist. Besonders bevorzugt ist die separate Dosierpumpe fluidisch vor, also stromaufwärts, der Dialyseflüssigkeitspumpe angeordnet. Die Dialyseflüssigkeitspumpe ist häufig als oszillierende Verdrängerpumpe ausgebildet, fördert also die Dialyseflüssigkeit nicht kontinuierlich. Für eine homogene Durchmischung der Konzentratflüssigkeit mit der in der Hauptleitung fließenden Flüssigkeit ist jedoch ein kontinuierlicher Fluss in der Hauptleitung wünschenswert. Daher ist die separate Dosierpumpe bevorzugt als kontinuierlich fördernde Dosierpumpe ausgebildet, insbesondere als quasi-kontinuierlich fördernde Dosierpumpe, beispielsweise als Zahnradpumpe. Durch die separate und kontinuierlich oder quasi-kontinuierlich fördernde Dosierpumpe wird eine volumenhomogene Mischung der Konzentratflüssigkeiten und des Dialysewassers sichergestellt.

Besonders bevorzugt sind zwei oder sogar mehr Konzentrateinleitungs-Anordnungen vorgesehen, die jeweils erfindungsgemäß passiv ausgebildet sind, also keine eigene Pumpe aufweisen. Zwischen der Dialysewasser-Quelle und der Hauptleitung ist ein Dialysewasser-Ventil angeordnet, an dem der Fluiddruck des Dialysewassers auf das erforderliche Maß gedrosselt wird. Das Dialysewasser-Ventil ist also ein Drosselventil.

In einer besonders bevorzugten Ausgestaltung kann das Dialysewasser-Ventil ein durch eine Gerätesteuerung steuerbares elektrisches Drosselventil sein, das bei Bedarf auch vollständig geschlossen werden kann.

Erfindungsgemäß wird die Konzentratflüssigkeit bzw. werden die Konzentratflüssigkeiten dem im Bereich der Einmündung in der Hauptleitung vorbeifließenden Dialysewasser zugemischt, indem das betreffende Konzentrat-Ventil, das von einer Gerätesteuerung elektrisch angesteuert wird, geöffnet wird. Gleichzeitig arbeitet die Dialyseflüssigkeits-Pumpe, so dass auf diese Weise in der Hauptleitung ein Unterdruck erzeugt wird, durch den die Flüssigkeit in der Hauptleitung zu der Dialysefiüssigkeits-Pumpe gesaugt wird. Aus dem Druckverhältnis zwischen dem von der Dialysewasser-Quelle kommenden Dialysewasser und der Konzentratflüssigkeit, die aus der Einleitungsleitung in die Einmündung fließt, bestimmt sich das tatsächliche Mischungsverhältnis des Dialysewassers mit der betreffenden Konzentratflüssigkeit. Das Druckverhältnis wird insbesondere auch durch das Dialysewasser-Ventil bestimmt, durch das das Dialysewasser von der Dialysewasser-Quelle gedrosselt in die Hauptleitung einfließt.

In einer besonders einfachen Ausführung ist also in dem Dialyseflüssigkeits-Strang, in dem die Dialyseflüssigkeit generiert wird, nur eine einzige Pumpe erforderlich und vorhanden, nämlich die Dialyseflüssigkeitspumpe, die die Dialyseflüssigkeit am fluidischen Ende der Hauptleitung zu dem Dialysator pumpt. Weitere Pumpen sind in dem Dialyseflüssigkeits-Strang nicht unbedingt erforderlich. Dadurch ist der Dlalyseflüssigkeits-Strang technisch sehr einfach aufgebaut, so dass die Gesamtkosten für das Hämodialysegerät auf diese Weise erheblich verringert werden können, und zwar umso mehr, je mehr Konzentrateinleitungs-Anordnungen dem Dialyseflüssigkeits-Strang zugeordnet sind.

Gemäß einer bevorzugten Ausführung ist das Konzentrat-Ventil in der Konzentrateinleitungs-Anordnung ein regelbares, besonders bevorzugt ein stufenlos regelbares Konzentrat-Ventil, das von der Gerätesteuerung entsprechend angesteuert wird. Durch ein graduelles Verstellen der Drosselung des regelbaren bzw. stufenlos regelbaren Konzentrat-Ventils kann das Druckverhältnis zwischen der in der Hauptleitung fließenden Flüssigkeit und der durch die Einmündung in die Hauptleitung einfließenden Konzentratflüssigkeit stufenlos eingestellt werden, so dass auf diese Weise das Mischungsverhältnis zwischen der in der Hauptleitung fließenden Flüssigkeit und der eingeleiteten Konzentratflüssigkeit genau eingestellt werden kann.

Besonders bevorzugt ist auch das Dialysewasser-Ventil als ein von der Gerätesteuerung steuerbares elektrisches Drosselventil ausgebildet, so dass die Drosselung durch das Dialysewasser-Ventil durch die Gerätesteuerung gesteuert bzw. jederzeit angepasst werden kann. Hierdurch kann in Zusammenwirkung mit der eingestellten Pumpleistung der Dialyseflüssigkeitspumpe ebenfalls das Mischungsverhältnis an der Einmündung zwischen der Flüssigkeit in der Hauptleitung und der eingeleiteten Konzentratflüssigkeit beeinflusst werden.

Vorzugsweise ist die Dialyseflüssigkeitspumpe ebenfalls stufenlos elektronisch regelbar, und ist hierzu mit der Gerätesteuerung elektrisch bzw. informell verbunden. Die Gerätesteuerung regelt und steuert also die Pumpleistung der Dialyseflüssigkeitspumpe, und steuert gegebenenfalls ebenfalls den Öffnungsgrad bzw. den Drosselgrad des Konzentrat-Ventils und/oder des Dialysewasser-Ventils, um auf diese Weise das Mischungsverhältnis und die Gesamt-Flussrate der zu dem Dialysator fließenden Dialyseflüssigkeit zu steuern bzw. zu regeln.

Gemäß einer bevorzugten Ausgestaltung weist die Konzentrateinleitungs-Anordnung fluidisch zwischen dem Konzentratflüssigkeits-Ventil und der Konzentratflüssigkeits-Einmündung einen Konzentratflüssigkeits-Drucksensor auf, der mit der Gerätesteuerung verbunden ist, die auch das Konzentratflüssigkeits-Ventil steuert. Alternativ oder ergänzend zu dem Konzentratflüssigkeits-Drucksensor kann auch ein Konzentratflüssigkeits-Flusssensor vorgesehen sein. Durch die Erfassung des Druckes und/oder des Flusses der Konzentratflüssigkeit kann indirekt oder direkt das Mischungsverhältnis zwischen der eingeleiteten Konzentratflüssigkeit und der in der Hauptleitung fließenden Flüssigkeit geregelt werden. Besonders bevorzugt ist hierzu in der Hauptleitung ebenfalls ein Drucksensor und/oder ein Flusssensor angeordnet, so dass aus dem Quotienten der betreffenden gemessenen Drücke und/oder Flüsse das Mischungsverhältnis genau bestimmt werden kann.

Gemäß einer besonders bevorzugten Ausgestaltung ist in der Hauptleitung stromabwärts der Konzentratflüssigkeits-Einleitung ein Leitfähigkeitssensor vorgesehen, der mit der Gerätesteuerung informell verbunden ist. Mit den von dem Leitfähigkeitssensor gelieferten Leitfähigkeits-Messwerten kann die Gerätesteuerung jedenfalls indirekt bzw. indiziell überprüfen, ob das über die Einstellung der Ventile angestrebte Mischungsverhältnis zwischen der Konzentratflüssigkeit und dem Dialysewasser tatsächlich erreicht wurde. Mit Hilfe des Leitfähigkeitssensors bzw. mehrerer Leitfähigkeitssensoren, die jeweils hinter jeder Konzentratflüssigkeits-Einleitung angeordnet sein können, kann ein geschlossener Regelkreis gebildet werden, so dass die Konzentration der Konzentratflüssigkeit in dem Dialysewasser geregelt werden kann. Hierdurch wird eine hohe Prozesssicherheit erlangt.

Gemäß einer bevorzugten Ausgestaltung ist in der Hauptleitung des Dialyseflüssigkeits-Strangs stromabwärts der betreffenden Konzentratflüssigkeits-Einleitung eine Mischkammer vorgesehen, In der das Dialysewasser mit der eingeleiteten Konzentratflüssigkeit homogen vermischt wird. Die Mischkammer ist dabei bevorzugt fluidisch vor einem entsprechenden Leitfähigkeitssensor angeordnet, wenn dieser vorgesehen ist. Durch die Mischkammer wird insbesondere das Messergebnis eines nachgeordneten Leitfähigkeitssensors aussagekräftiger, da große temporäre Sprünge der gemessenen Konzentration bzw. Leitfähigkeit auf diese Weise vermieden werden.

Gemäß einer bevorzugten Ausgestaltung ist die Konzentratflüssigkeits-Einmündung als sogenannter Venturi-Mischer ausgebildet. Ein Venturi-Mischer bietet eine gute homogene Durchmischung der eingeleiteten Konzentratflüssigkeit in den Dialysewasser-Hauptstrom.

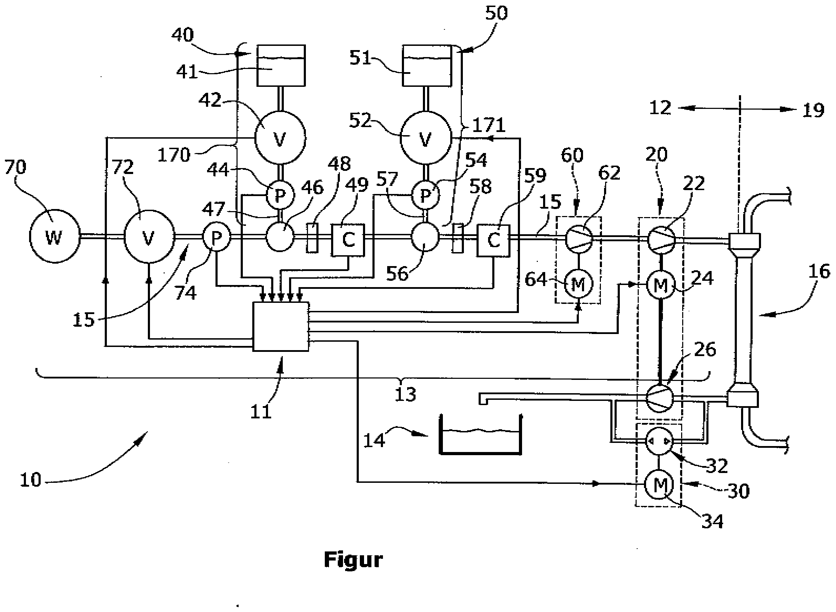

Im Folgenden wird unter Bezugnahme auf die Zeichnung ein Ausführungsbeispiel der Erfindung näher erläutert.

Die Figur zeigt schematisch ein Hämodialysegerät mit einem detailliert dargestellten Dialyseflüssigkeits-Strang, in dem eine Dialyseflüssigkeit generiert wird.

In der Figur ist schematisch ein Hämodialysegerät 10 dargestellt, in dem aus Dialysewasser und zwei Konzentratflüssigkeiten 41,51 in einem Dialyseflüssigkeits-Strang 13 eine Dialyseflüssigkeit generiert wird, die einem Dialysator 16 durch eine Dialyseflüssigkeitspumpe 22 zugeführt wird. Von dem Dialysator 16 aus fließt das Dialysat, also die frühere Dialyseflüssigkeit, nachdem sie den Dialysator 16 durchflossen hat, in einen Abfalltank 14. Hierzu sind stromabwärts des Dialysators 16 eine Abfallpumpe 26 und eine zu der Abfallpumpe 26 fluidisch parallel angeordnete bidirektionale Ultrafiltrationspumpe 30 vorgesehen, die das Dialysat in der Regel in Richtung des Abfalltanks 14 pumpen. Die Ultrafiltrationspumpe 30 weist ein Pumpwerk 32 auf, das durch einen elektrischen Antriebsmotor 34 angetrieben wird.

Der Dialysator 16 weist eine nicht dargestellte Dialysatormembran auf, die eine Blutseite 19 von einer Dialysatseite 12 trennt. Der Dialyseflüssigkeits-Strang 13, die Dialysatkammer des Dialysators 16, die Abfallpumpe 26, die Ultrafiltrationspumpe 30 und der Abfalltank 14 sind auf der Dialysatseite 12 angeordnet.

Die Dialyseflüssigkeitspumpe 22 und die Abfallpumpe 26 sind vorliegend mechanisch miteinander gekoppelt, und bilden auf diese Weise gemeinsam ein Bilanzierwerk 20. Die Dialyseflüssigkeitspumpe 22 und die Abfallpumpe 26 sind beide als oszillierende Verdrängerpumpen ausgebildet und werden durch einen einzigen Antriebsmotor 24 gemeinsam mechanisch angetrieben.

Stromabwärts der Dialyseflüssigkeitspumpe 20 ist eine separate Dosierpumpe 60 angeordnet, die aus einem Pumpwerk 62 und einem elektrischen Antriebsmotor 64 besteht. Die Dosierpumpe 60 bzw. das Pumpwerk 62 ist vorliegend als quasi-kontinuierliche Zahnradpumpe ausgebildet.

In dem Dialyseflüssigkeits-Strang 13 wird aus Dialysewasser, das von einer Dialysewasser-Quelle 70 kommt, durch Hinzumischen der Konzentratflüssigkeiten 41,51 die Dialyseflüssigkeit generiert. Es handelt sich vorliegend um eine erste Konzentratflüssigkeit 41, die eine Bikarbonat-Lösung ist, und um eine zweite Konzentratflüssigkeit 51, die ein Säurekonzentrat ist.

Der Dialyseflüssigkeits-Strang 13 weist eine Hauptleitung 15 auf, die sich von der Dialysewasser-Quelle 70 bis zum Dialysator 16 erstreckt. Dem in der Hauptleitung 15 fließenden Dialysewasser wird an zwei im Verlauf der Hauptleitung 15 angeordneten Einmündungen 46,56 jeweils eine Konzentratflüssigkeit 41,51 zugemischt, so dass das Gesamtgemisch schließlich die Dialyseflüssigkeit ergibt, die durch die Dosierpumpe 60 und die Dialyseflüssigkeits-Pumpe 22 zu dem Dialysator 16 gepumpt wird.

Von der Dialysewasser-Quelle 70 aus stromabwärts gesehen weist die Hauptleitung 15 zunächst ein Dialysewasser-Ventil 72 auf, das als ein stufenlos regelbares Drosselventil ausgebildet ist, das auch vollständig verschlossen werden kann. Stromabwärts des Dialysewasser-Ventils 72 ist ein Dialyseflüssigkeits-Drucksensor 74 vorgesehen, durch den der Fluiddruck an dieser Stelle der Hauptleitung 15 gemessen wird. Stromabwärts des Dialyseflüssigkeits-Drucksensors 74 Ist eine erste Konzentratflüssigkeits-Einmündung 46 vorgesehen, die besonders bevorzugt als Venturi-Mischer ausgebildet ist.

An der ersten Konzentratflüsslgkeits- Einmündung 46 wird die erste Konzentratflüssigkeit 41 in das Dialysewasser eingeleitet. Hinter der ersten Konzentratflüssigkeits-Einmündung 46 sind eine erste Mischkammer 48 und fluidisch dahinter ein erster Leitfähigkeitssensor 49 angeordnet. In der Mischkammer 48 wird die zuvor eingeleitete Konzentratflüssigkeit homogen mit dem Dialysewasser vermischt, so dass der erste Leitfähigkeitssensor 49 ein sprungfreies und ruhiges Konzentrationssignal generiert. Stromabwärts des ersten Leitfähigkeitssensors 49 ist die zweite Konzentratflüssigkelts-Einmündung 56 angeordnet, hinter der eine zweite Mischkammer 48 und ein zweiter Leitfähigkeitssensor 59 vorgesehen sind.

Der Dialyseflüssigkeits-Strang 13 weist also zwei Konzentrateinleitungs-Anordnungen 170,171 auf, durch die die betreffende Konzentratflüssigkeit 41,51 in die Konzentratflüsslgkelts-Einmündungen 46,56 eingespeist wird. Jede Konzentrateinleitungs-Anordnung 170,171 weist einen Konzentratflüssigkeits-Tank 40,50 auf, hinter dem fluidisch ein schaltbares und stufenlos regelbares Konzentrat-Ventil 42,52 angeordnet ist. Stromabwärts des Konzentrat-Ventils 42,52 ist jeweils ein Konzentratflüssigkeits-Drucksensor 44,54 angeordnet. Zwischen dem Konzentratflüssigkeits-Behälter 40,50 und der Konzentratflüssigkeits-Einmündung 46,56 verläuft jeweils eine Einleitung-Leitung 47,57, die in die Konzentratflüssigkeits-Einmündung 46,56 mündet.

Das Hämodialysegerät 10 weist eine Gerätesteuerung 11 auf, die über entsprechende Steuerleitungen die Antriebsmotoren 24,34,64 der Pumpen 20,30,60 sowie die Ventile 42,52,72 ansteuert. Ferner weist die Gerätesteuerung 11 mehrere Sensoreingänge auf, über die sie mit den Drucksensoren 44,54,74 und den Leitfähigkeitssensoren 49,59 informell bzw. elektrisch verbunden ist.

In der Gerätesteuerung 11 ist jeweils eine Soll-Konzentration für die erste Konzentratflüssigkeit 41 und für die zweite Konzentratflüssigkeit 51 im Verhältnis zu dem Dialysewasser abgespeichert. Zum Generieren der Dialyseflüssigkeit werden die Dialyseflüssigkeits-Pumpe 22 und die Dosierpumpe 60 in Betrieb genommen. Ferner werden die beiden Konzentratflüssigkeits-Ventile 42,52 sowie das Dialysewasser-Ventil 72 derart geöffnet bzw. gedrosselt, dass sich aus den hierdurch verursachten Druckverhältnissen voraussichtlich die jeweilige Soll-Konzentration für die erste und die zweite Konzentratflüssigkeit ergibt. Die tatsächliche Ist-Konzentration wird jeweils durch die beiden Leitfähigkeits-Sensoren 49,59 ermittelt und einer entsprechenden Regelung in der Gerätesteuerung 11 zugeführt, die, soweit erforderlich, durch eine Veränderung der Drosselung der stufenlos einstellbaren Ventile 42,52,72 korrigierend eingreift.

In den beiden Konzentrateinleitungs- Anordnungen 170,171 ist jeweils keine Pumpe vorgesehen.

Die Konzentratventile 42, 52 können bevorzugt als stufenlos einstellbare Schlauch-Quetschventile ausgebildet sein. Die Abquetscheinrichtung kann nach Art einer Schnecke ausgebildet sein.

## Patentansprüche

1. Hämodialysegerät (10) mit einem Dialyseflüssigkeits-Strang (13), in dem eine Dialyseflüssigkeit generiert wird, wobei der Dialyseflüssigkeits-Strang (13) aufweist:
ein Dialysewasser-Ventil (72), durch das das Dialysewasser von einer Dialysewasser-Quelle (70) in eine Hauptleitung (15) fließen kann,
eine Dialyseflüssigkeitspumpe (22), die Dialyseflüssigkeit von der Hauptleitung (15) zu einem Dialysator (16) pumpt, und
einer Konzentratelnleitungs-Anordnung (170,171) mit einem Konzentratflüssigkeits- Behälter (40,50), einer Konzentratflüssigkeit (41,51) in dem Behälter (40,50) und einer Konzentratflüssigkeits-Einmündung (46,56), an der eine von dem Konzentratflüssigkeits-Behälter (40,50) kommende Einleitungsleitung (47,57) in die Hauptleitung (15) mündet,
wobei die Konzentrateinleitungs-Anordnung (170,171) ein schaltbares Konzentrat-Ventil (42,52) aufweist, und
wobei die Konzentrateinleitungs-Anordnung (170,171) keine Pumpe aufweist,
**dadurch gekennzeichnet,**
**dass** der Dialyseflüssigkeits-Strang (13) fluidisch zwischen der Konzentratflüssigkeits-Einmündung (46,56) und der Dialyseflüssigkeits-Pumpe (22) eine separate Dosierpumpe (60) aufweist.

2. Hämodialysegerät (10) nach Anspruch 1, wobei das Konzentrat-Ventil (42,52) ein regelbares, besonders bevorzugt ein stufenlos regelbares Konzentrat-Ventil (42,52) ist.

3. Hämodialysegerät (10) nach einem der vorangegangenen Ansprüche, wobei das Dialysewasser-Ventil (72) ein Drosselventil Ist, und bevorzugt ein durch eine Gerätesteuerung (11) steuerbares elektrisches Drosselventil ist.

4. Hämodialysegerät (10) nach einem der vorangegangenen Ansprüche, wobei das Dialysewasser-Ventil (72) als ein schaltbares Dialysewasser-Ventil (72) ausgebildet ist, insbesondere als ein stufenlos regelbares Dialysewasser-Ventil (72).

5. Hämodialysegerät (10) nach einem der vorangegangenen Ansprüche, wobei die Konzentrateinleitungs-Anordnung (170,171) fluidisch zwischen dem Konzentratflüssigkeits-Ventil (42,52) und der Konzentratflüssigkeits-Einmündung (46,56) einen Konzentratflüssigkeits- Drucksensor (44,54) aufweist, der mit einer Gerätesteuerung (11) verbunden ist, die das Konzentrationsflüssigkeits-ventil (42,52) steuert.

6. Hämodialysegerät (10) nach einem der vorangegangenen Ansprüche, wobei der Dialyseflüssigkeits-Strang (13) zwischen dem Dialysewasser-Ventil (72) und der Konzentratflüssigkeits-Einmündung (46,56) einen Dialyseflüssigkeits-Drucksensor (74) aufweist, der mit einer Gerätesteuerung (11) verbunden ist, die das Dialysewasser-Ventil (72) und das Konzentrationsflüssigkeits-Ventil (42,52) steuert,

7. Hämodialysegerät (10) nach Anspruch 1, wobei die Dosierpumpe (60) als kontinuierlich oder quasi-kontinuierlich fördernde Dosierpumpe (60) ausgebildet ist, besonders bevorzugt als Verdrängerpumpe.

8. Hämodialysegerät (10) nach einem der vorangegangenen Ansprüche, wobei der Dialyseflüssigkeits-Strang (13) stromabwärts der Konzentratflüssigkeits-Einleitung (46,56) einen Leitfähigkeitssensor (49,59) aufweist, der mit einer Gerätesteuerung (11) verbunden ist, die das Konzentratflüssigkeits-Ventil (42,52) steuert.

9. Hämodialysegerät (10) nach einem der vorangegangenen Ansprüche, wobei der Dialyseflüssigkeits-Strang (13) stromabwärts der Konzentratflüssigkeits-Einleitung (46,56) im Verlauf der Hauptleitung (15) eine Mischkammer (48,58) aufweist.

10. Hämodialysegerät (10) nach einem der vorangegangenen Ansprüche, wobei die Konzentratflüssigkeits-Einmündung (46,56) als Venturi-Mischer ausgebildet ist.

11. Verfahren zur Generierung einer Dialyseflüssigkeit mit einem Hämodialysegerät (10) mit den Merkmalen einer der vorangegangenen Ansprüche, mit den Verfahrensschritten:
Öffnen des Konzentratflüssigkeits-Ventils (42,52), und
Aktivieren der Dialysatflüssigkeits-Pumpe (22), und der Dosierpumpe (60),
so dass sowohl Dialysewasser aus der Dialysewasser-Quelle (70) als auch Konzentratflüssigkeit (41,51) aus der Konzentrateinleitungs-Anordnung durch die Hauptleitung (15) zu der Dialysatflüssigkeits-Pumpe (22) gepumpt werden.

## Claims

1. Hemodialysis apparatus (10) having a dialysis liquid system (13) in which a hemodialysis liquid is produced, the dialysis liquid system (13) comprising:
a dialysis water valve (72) through which the dialysis water can flow from a dialysis water source (70) into a main conduit (15),
a dialysis liquid pump (22) which pumps the dialysis liquid from the main conduit (15) to a dialyzer (16), and
a concentrate introduction unit (170, 171) with a concentrate liquid container (40, 50), a concentrate liquid (41, 51) in the container (40, 50) and a concentrate liquid inflow (46, 56) where an introduction conduit (47, 57) from the concentrate liquid container (40, 50) opens into the main conduit (15),
wherein the concentrate introduction unit (170, 171) comprises a switchable concentrate valve (42, 52), and
wherein the concentrate introduction unit (170, 171) comprises no pump,
**characterized in that**
the dialysis liquid system (13) comprises a separate dosing pump (60) fluidically between the concentrate liquid inflow (46, 56) and the dialysis liquid pump (22).

2. Hemodialysis apparatus (10) of claim 1, wherein the concentrate liquid valve (42, 52) is a controllable, particularly preferred a variably controllable concentrate valve (42, 52).

3. Hemodialysis apparatus (10) of one of the preceding claims, wherein the dialysis water valve (72) is a throttle valve and preferably an electric throttle valve controllable by an apparatus control (11).

4. Hemodialysis apparatus (10) of one of the preceding claims, wherein the dialysis water valve (72) is configured as a switchable dialysis water valve (72), in particular a variably controllable dialysis water valve (72).

5. Hemodialysis apparatus (10) of one of the preceding claims, wherein the concentrate introduction unit (170, 171) comprises a concentrate liquid pressure sensor (44, 54) fluidically between the concentrate liquid valve (42, 52) and the concentrate liquid inflow (46, 56), the sensor being connected to an apparatus control (11) that controls the concentrate liquid valve (42, 52).

6. Hemodialysis apparatus (10) of one of the preceding claims, wherein the dialysis liquid system (13) comprises a dialysis water pressure sensor (74) between the dialysis water valve (72) and the concentrate liquid inflow (40, 50), the sensor being connected to an apparatus control (11) that controls the dialysis water valve (72) and the concentrate liquid valve (42, 52).

7. Hemodialysis apparatus (10) of claim 1, wherein the dosing pump (60) is designed as a continuously or quasi-continuously conveying dosing pump (60), particularly preferred as a positive displacement pump.

8. Hemodialysis apparatus (10) of one of the preceding claims, wherein the dialysis liquid system (13) comprises a conductivity sensor (49, 59) downstream of the concentrate liquid inflow (46, 56), the sensor being connected to an apparatus control (11) that controls the concentrate liquid valve (42, 52).

9. Hemodialysis apparatus (10) of one of the preceding claims, wherein the dialysis liquid system (13) comprises, in the course of the main conduit (15), a mixing chamber (48, 58) downstream of the concentrate liquid inflow (46, 56).

10. Hemodialysis apparatus (10) of one of the preceding claims, wherein the concentrate liquid inflow (46, 56) is designed as a Venturi mixer.

11. Method for producing a dialysis liquid using a hemodialysis apparatus (10) with the features of one of the preceding claims, the method comprising the following steps:
opening the concentrate liquid valve (42, 52), and
activating the dialysate liquid pump (22) and the dosing pump (50),
so that both dialysis water from the dialysis water source (70) and concentrate liquid (41, 51) from the concentrate introduction unit are pumped through the main conduit (15) to the dialysate liquid pump (22).

## Revendications

1. Appareil d'hémodialyse (10) avec un système de liquide de dialyse (13), dans lequel un liquide de dialyse est produit, le système de liquide de dialyse (13) comportant:
une soupape d'eau de dialyse (72) permettant l'écoulement de l'eau de dialyse à partir de la source d'eau de dialyse (70) dans une conduite principale (15),
une pompe de liquide de dialyse (22) pompant de liquide de dialyse de la conduite principale (15) vers un dialyseur (16), et
un ensemble d'introduction de concentré (170, 171) avec un récipient de liquide de concentré (40, 50), un liquide de concentré (41, 51) dans le récipient (40, 50) et une embouchure de liquide de concentré (46, 56) où la conduite d'introduction (47, 57) venant du récipient d'eau de dialyse s'ouvre dans la conduite principale (15),
ledit ensemble d'introduction de concentré (170, 171) comprenant une soupape de concentré (42, 52) commutable,
ledit ensemble d'introduction de concentré (170, 171) ne comportant pas une pompe,
**caractérisé en ce que**
le système de liquide de dialyse (13) comprend une pompe de dosage (60) séparée, la pompe étant disposée fluidiquement entre l'embouchure de liquide de concentré (46, 56) et la pompe de liquide de dialyse (22).

2. Appareil d'hémodialyse (10) selon la revendication 1, dans lequel la soupape de liquide de concentré (42, 52) est une soupape réglable, de façon particulièrement préférée une soupape réglable en continu.

3. Appareil d'hémodialyse (10), selon l'une quelconque des revendications précédentes, dans lequel la soupape d'eau de dialyse (72) est une soupape d'étranglement et, de préférence, une soupape d'étranglement électrique apte à être commandée par une commande d'appareil (11).

4. Appareil d'hémodialyse (10), selon l'une quelconque des revendications précédentes, dans lequel la soupape d'eau de dialyse (72) est conçue comme une soupape d'eau de dialyse (72) commutable, à savoir une soupape d'eau de dialyse (72) réglable en continu.

5. Appareil d'hémodialyse (10), selon l'une quelconque des revendications précédentes, dans lequel l'ensemble d'introduction de concentré (170, 171) comprend un capteur de pression de liquide de concentré (42, 52) fluidiquement entre la soupape de liquide de concentré (42, 52) et l'embouchure de liquide de concentré (46, 56), le capteur étant relié avec une commande d'appareil (11) commandant la soupape de liquide de concentré (42, 52).

6. Appareil d'hémodialyse (10), selon l'une quelconque des revendications précédentes, dans lequel le système de liquide de dialyse (13) comprend un capteur de pression de liquide de dialyse (74) entre la soupape d'eau de dialyse (72) et l'embouchure de liquide de concentré (46, 56), le capteur étant relié avec une commande d'appareil (11) commandant la soupape d'eau de dialyse (72) et la soupape de liquide de concentré (42, 52).

7. Appareil d'hémodialyse (10) selon la revendication 1, dans lequel la pompe de dosage (60) est conçue comme une pompe de dosage (60) convoyant en continu ou quasi-continu, de façon particulièrement préférée comme une pompe à déplacement positif.

8. Appareil d'hémodialyse (10), selon l'une quelconque des revendications précédentes, dans lequel le système de liquide de dialyse (13) comprend un capteur de conductivité (49, 59) en aval de l'embouchure de liquide de concentré (46, 56), le capteur étant relié avec une commande d'appareil (11) commandant la soupape de liquide de concentré (42, 52).

9. Appareil d'hémodialyse (10), selon l'une quelconque des revendications précédentes, dans lequel, en aval de l'embouchure de liquide de concentré (46, 56), le système de liquide de dialyse (13) comprend une chambre de mélange (48, 58) sur le parcours de la conduite principale (15).

10. Appareil d'hémodialyse (10), selon l'une quelconque des revendications précédentes, dans lequel l'embouchure de liquide de concentré (46, 56) est conçu comme un mélangeur Venturi.

11. Procédé de production d'un liquide de dialyse avec un appareil de dialyse (10) avec les caractéristiques de l'une quelconque des revendications précédentes, avec les étapes suivantes:
ouvrir la soupape de liquide de concentré (42, 52),
activer la pompe de liquide de dialyse (22) et la pompe de dosage (60),
de sorte que de l'eau de dialyse soit pompée à partir de la source d'eau de dialyse (70) et de liquide de concentré (41, 51) soit pompé à partir de l'ensemble d'introduction de concentré vers la pompe de liquide de dialyse (22) à travers la conduite principale (15.
